# EUROPÄISCHE PATENTSCHRIFT

(11) **EP 0 478 026 B1**
(45) Veröffentlichungstag und Bekanntmachung des Hinweises auf die Patenterteilung: **05.06.1996**
(21) Anmeldenummer: 91202117.7
(22) Anmeldetag: 20.08.1991
(51) Int. Cl.: A61B 5/00

(54) **Verfahren zur Erfassung von Anomalien der Haut, insbesondere von Melanomen, sowie Vorrichtung zur Durchführung des Verfahrens**
Method and device for aquisioned anomalies of the skin, particulary of malanomen
Méthode et dispositif pour l'aquisition d'anomalies de la peau, en particulier les mélanomen

(30) Priorität: 24.08.1990 DE 4026821
(43) Veröffentlichungstag der Anmeldung: 01.04.1992
(73) Patentinhaber: Philips Patentverwaltung GmbH, 22335 Hamburg (DE); Philips Electronics N.V., 5621 BA Eindhoven (NL)
(72) Erfinder: Martens, Gerhard, Dr., W-2359 Henstedt-Ulzburg (DE); Günzel, Erhard, W-2725 Westerwalsede (DE)
(74) Vertreter: Hartmann, Heinrich, Dipl.-Ing.

(56) Entgegenhaltungen:
- EP-A- 0 003 015
- WO-A-88/09145
- US-A- 3 973 129
- US-A- 4 505 583

## Beschreibung

Die Erfindung betrifft ein Verfahren nach dem Oberbegriff des Anspruchs 1 sowie eine Vorrichtung zur Durchführung dieses Verfahrens. Ein solches Verfahren und eine solche Anordnung sind aus der DE-A-37 18 202 bekannt. Bei der bekannten Vorrichtung wird mittels eines Spektralphotometers das rückgestreute Licht aufgenommen und es werden die Intensitäten des reflektierten Lichts und des Fluoreszenzlichts ermittelt. Da das Fluoreszenzlicht im Übergangsbereich zwischen Melanom und gesundem Gewebe besonders intensiv ist, kann aus dem so bestimmten Spektrum mittels einer empirisch ermittelten, gespeicherten Tabelle abgeleitet werden, ob sich in dem vom Spektralphotometer jeweils erfaßten Bezirk der Gewebeoberfläche eine Anomalie befindet oder nicht.

Melanome sind unregelmäßig geformte und unregelmäßig pigmentierte Läsionen der Haut, die äußerlich sehr einem Muttermal (Naevus) ähneln. Manche Patienten haben auf ihrer gesamten Körperoberfläche relativ viele Muttermale. Wollte man mit Hilfe des bekannten Verfahrens untersuchen, ob es sich dabei tatsächlich nur um Muttermale und nicht um Melanome handelt, müßte man das Spektralphotometer relativ zu dem zu untersuchenden Patienten in eine Vielzahl von Positionen bringen und die vom Spektralphotometer ermittelten Intensitäten auswerten. Eine solche Untersuchung wäre sehr zeitaufwendig.

Aufgabe der vorliegenden Erfindung ist es, ein Verfahren der eingangs genannten Art so auszugestalten, daß die Untersuchung eines ausgedehnten Bereiches verhältnismäßig schnell erfolgen kann. Ausgehend von einer Anordnung zur Durchführung des Verfahrens wird diese Aufgabe durch die im Anspruch 1 angegebenen Maßnahmen gelöst. Als "Fluoreszenzbild" wird dabei die Abbildung des Untersuchungsbereichs in einem Wellenlängenbereich bezeichnet, der größere Wellenlängen umfaßt als der erste Wellenlängenbereich.

Während also bei dem bekannten Verfahren eine Auswertung des spektralen Verlaufs des rückgestreuten Lichts erfolgt, was naturgemäß immer nur für einen kleinen Bezirk der Haut erfolgen kann, erfolgt bei der Erfindung in einem ausgedehnten Bereich (d.h. einen Bereich von 50 mm x 50 mm oder mehr) eine Auswertung des räumlichen Verlaufs der Intensität - vorzugsweise in dem Wellenlängenbereich, in dem die Fluoreszenz ihr Maximum aufweist. Dadurch lassen sich mehrere Läsionen bzw. Muttermale auf einem großen Hautareal (z.B. Rücken) gleichzeitig untersuchen.

Die Auswertung des Fluoreszenzbildes allein ist noch nicht hinreichend zuverlässig, weil auch die Topologie der Hautoberfläche im Untersuchungsbereich einen sehr starken Einfluß auf die Intensitätsverteilung des Fluoreszenzbildes hat. Ein Intensitätsmaximum im Fluoreszenzbild ist somit noch kein eindeutiger Hinweis auf das Vorliegen einer Anomalie, weil die Intensitätserhöhung in dem Fluoreszenzbild um eine Anomalie herum kleiner sein kann als die von der Tropologie und anderen Einflüssen bewirkte Intensitätsänderung.

Deshalb wird bei der Erfindung ein Referenzbild erzeugt. Das Referenzbild ist ein Bild, das von den Intensitätsunterschieden der Fluoreszenz zumindest weitgehend unabhängig ist, jedoch sämtliche anderen Einflüsse erfährt, die auch auf das Fluoreszenzbild einwirken. Erzeugt man daher durch Normierung des Fluoreszenzbildes auf das Referenzbild ein Ausgangsbild, dann sind in diesem Ausgangsbild die letztgenannten Einflüsse praktisch vollständig eliminiert. Eine gesunde Hautoberfläche wird daher in diesem Ausgangsbild nahezu gleichmäßig hell abgebildet, während der ca. 10 mm breite Umgebungsbereich eines Melanoms in dem Ausgangsbild eine erhöhte Intensität aufweist.

Es sei an dieser Stelle erwähnt, daß aus der US-PS 4,236,082 ein Identifikationsverfahren bekannt ist, bei dem die Hand eines Menschen kurzzeitig von einer UV-Lichtquelle bestrahlt wird und bei dem eine Kamera ein Fluoreszenzbild der Hand aufnimmt. Mit diesem Verfahren soll der Kontrast von feinen Strukturen der Hand, z.B. der Handlinie, vergrößert werden.

Eine Vorrichtung zur Durchführung des erfindungsgemäßen Verfahrens mit einer Lichtquelle ist gekennzeichnet durch wenigstens eine Lichtquelle zur Beleuchtung des Untersuchungsbereichs mit Licht in einem ersten und in einem zweiten Wellenlängenbereich, eine auf den Untersuchungsbereich ausrichtbaren Kamera zum Aufnehmen des Fluoreszenzbildes und des Referenzbildes und zur Erzeugung entsprechender elektrischer Signale, eine Speicheranordnung zum Speichern wenigstens eines Bildes und eine Verarbeitungseinheit zur Erzeugung eines Ausgangsbildes in Abhängigkeit von dem Quotienten der denselben Bildpunkten zugeordneten Signalwerte des Fluoreszenz- und des Referenzbildes.

Da der zweite Wellenlängenbereich in der Regel nicht mit dem ersten Wellenlängenbereich zusammenfällt, kann man die für die Aufnahme des Fluoreszenzbildes bzw. des Referenzbildes erforderlichen Beleuchtung mit Hilfe zweier Lichtquellen erzeugen, die je einen der beiden Wellenlängenbereiche emittieren. Da das Referenzbild aber bis auf die unterschiedlich starke Fluoreszenz von den gleichen Faktoren abhängen muß wie das Fluoreszenzbild, müßten die Lichtquellen beim Aufnehmen der Bilder dieselbe Position und darüberhinaus dieselbe Richtcharakteristik usw. aufweisen. Eine günstigere Lösung ist dem demgegenüber gekennzeichnet durch mindestens eine sowohl im ersten als auch im zweiten Wellenlängenbereich emittierende Lichtquelle, ein erstes Filter zum Durchlassen von Licht aus dem ersten Wellenlängenbereich und zum Unterdrücken von Licht mit der Wellenlänge des Fluoreszenzlichts sowie durch ein zweites Filter zum Durchlassen des zweiten Wellenlängenbereiches, wobei jeweils eines der beiden Filter in den Strahlengang zwischen der Lichtquelle und dem Untersuchungsbereich anordbar ist.

Bei dieser Ausgestaltung der Erfindung ist nur eine Lichtquelle vorhanden, so daß bei beiden Bildern dieselbe Lage zwischen Lichtquelle und Untersuchungsbereich, die gleiche Richtcharakteristik usw. gewährleistet ist. Da lediglich die beiden Filter im Strahlengang ausgewechselt werden müssen, können Fluoreszenzbild und Referenzbild in dichtem zeitlichen Abstand erzeugt werden, so daß Bewegungsartefakte weitgehend vermieden werden. Es können auch mehrere Lichtquellen benutzt werden, die Licht in beiden Wellenlängen emittieren und die vorzugsweise gleichmäßig um die Kamera herum verteilt sind und den Untersuchungsbereich gleichmäßig beleuchten.

Eine Weiterbildung der Erfindung ist gekennzeichnet durch eine im Strahlengang der Lichtquelle befindliche Mattscheibe. Dadurch ergibt sich eine symmetrische Strahlungscharakteristik und eine gleichmäßige Beleuchtung des Untersuchungsbereichs.

Eine Weiterbildung der Erfindung enthält ein vor der Kamera anordbares Filter zum Durchlassen von Licht aus dem zweiten Wellenlängenbereich. Dieses beim Aufnehmen des Referenzbildes im Strahlengang der Kamera befindliche Filter unterdrückt Licht aus dem ersten Wellenlängenbereich und dämpft etwaiges Umgebungslicht, so daß auch in einem nicht vollständig abgedunkelten Untersuchungsraum gearbeitet werden kann. Gegebenenfalls können der zweite Wellenlängenbereich und der Wellenlängenbereich des Fluoreszenzlichts zusammenfallen; in diesem Fall kann das Filter auch bei der Aufnahme des Fluoreszenzbildes im Strahlengang verbleiben.

In weiterer Ausgestaltung der Erfindung sind in den Strahlengang vor der Kamera und der Lichtquelle einführbare Polarisationsfilter, die so beschaffen und angeordnet sind, daß die gerichtete Reflexion in dem von der Kamera erzeugten Bild unterdrückt wird, vorgesehen. Die Polarisationsfilter werden bei der Aufnahme des Referenzbildes benutzt und unterdrücken die gerichtete Reflexion (Glanzreflexion). Das Referenzbild ist also nur ein Streubild des Untersuchungsbereichs.

Die Erfindung wird nachstehend anhand der Zeichnung näher erläutert. Es zeigen
Fig. 1 eine Vorrichtung zur Durchführung des erfindungsgemäßen Verfahrens,
Fig. 2a ein Helligkeitsprofil im Fluoreszenzbild und im Referenzbild für die gleiche Profillinie und
Fig. 2b das zugehörige Profil im Ausgangsbild.

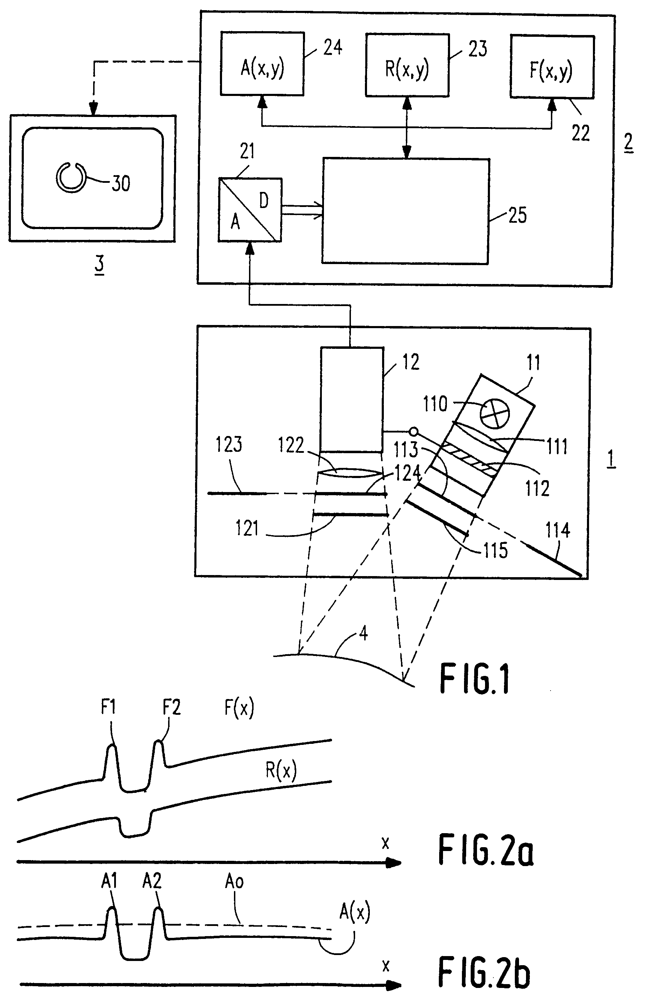

Die in Fig. 1 dargestellte Vorrichtung umfaßt eine Bilderzeugungseinrichtung 1, eine Bildverarbeitungseinheit 2 und eine Bildwiedergabeeinheit 3. Die Bilderzeugungseinheit 1 erzeugt von einem Untersuchungsbereich 4 (der Haut eines Patienten) Fluoreszenzbilder und Referenzbilder, die von der Bildverarbeitungseinheit 2 zu einem Ausgangsbild verarbeitet werden, das auf der Bildwiedergabeeinheit 3 dargestellt werden kann.

Die Bilderzeugungseinrichtung umfaßt ein Gehäuse bzw. eine Halterungseinrichtung 10, das bzw. die in einem nicht näher dargestellten Stativ bewegbar und schwenkbar angeordnet ist. In dem Gehäuse bzw. der Halterungseinrichtung befinden sich eine Beleuchtungseinrichtung 11 und eine Videokamera 12 in einer definierten Position zueinander und zum Gehäuse. Die Beleuchtungseinrichtung 11 beleuchtet den Untersuchungsbereich 4 bei der Aufnahme eines Fluoreszenzbildes mit ultraviolettem Licht in einem Wellenlängenbereich von 340 bis 380 nm. Bei der Aufnahme eines Referenzbildes wird blaues Licht in einem Wellenlängenbereich um 435 nm herum erzeugt und der Lichtdurchgang für das UV-Licht gesperrt.

Die Beleuchtungseinrichtung umfaßt eine Lichtquelle 110, die Licht in einem Wellenlängenbereich erzeugen kann, der die genannten Bereiche einschließt. Geeignete Lichtquellen sind beispielsweise Metallhalogen-UV-Lampen oder UVA-Leuchtstoffröhren, wie sie in Oberkörper- oder Ganzkörperbräunungsgeräten benutzt werden. Nach einer Projektionsoptik 111 durchsetzt das von der Lichtquelle erzeugte Licht eine Mattscheibe 112, die eine symmetrische Strahlungsverteilung bzw. eine gleichmäßige Beleuchtung des Untersuchungsbereichs bewirkt.

Die Beleuchtungseinrichtung 11 umfaßt ferner zwei Filter 113 bzw. 114, von denen sich jeweils eines im Strahlengang hinter der Mattscheibe 112 befindet und die über einen Filterwechsler miteinander gekoppelt sein können. Das Filter 113, das sich bei der Erzeugung eines Referenzbildes im Strahlengang befindet, unterdrückt ultraviolettes Licht und läßt sichtbares Licht durch. Hingegen läßt der Filter 114 ultraviolettes Licht durch und unterdrückt Licht zumindest im Fluoreszenz-Wellenlängenbereich (450 bis 490 nm).

Außerdem ist bei der Aufnahme eines Referenzbildes im Strahlengang der Beleuchtungseinrichtung 11 noch ein Polarisationsfilter 115 vorhanden, das in Verbindung mit einem entsprechenden Polarisationsfilter 121 vor der Kamera 12 die gerichtete Reflexion bzw. die Glanzreflexion unterdrückt. Vorzugsweise sind die Filter 113 und 115 in einer gemeinsamen Fassung untergebracht. Einige Polarisationsfilter sind für ultraviolettes Licht nahezu undurchlässig. In diesen Fällen kann auf das UV-Sperrfilter 113 verzichtet werden; das Polarisationsfilter 115 muß dann aber bei der Aufnahme eines Fluoreszenzbildes aus dem Strahlengang entfernt werden.

Vor der Kamera 12, die ein von der Helligkeit an den verschiedenen Bildpunkten linear abhängiges elektrisches Videosignal erzeugt, das dem Bild auf ihrer lichtempfindlichen Fläche entspricht, befindet sich ein Kameraobjektiv 122, das den Untersuchungsbereich 4 auf die lichtempfindliche Fläche der Kamera projiziert. Außerdem sind zwei Filter 123 und 124 vorgesehen, von denen sich jeweils einer im Strahlengang befindet. Das Filter 124 hat einen schmalen Durchlaßbereich um etwa 435 nm, während das Filter 123 einen Durchlaßbereich von 450 bis 490 nm hat. Wie durch eine gestrichelte Linie angedeutet, können die Filter mittels eines Filterwechslers gewechselt werden. Das Polarisationsfilter 121 ist vorzugsweise mit in die Fassung des Filters 124 montiert.

Das Ausgangssignal der Kamera 12 wird einem in der Bildverarbeitungseinrichtung 2 enthaltenen Analog-Digital-Wandler 21 zugeführt, der daraus eine Folge von digitalen Datenworten erzeugt, die die Helligkeit jeweils an einem Bildpunkt des von der Kamera 12 aufgenommenen Bildes darstellen. Die Bildverarbeitungseinheit 2 enthält drei Bildspeicher 22, 23 und 24 sowie einen Bildverarbeitungsprozessor 25.

Bei der Aufnahme eines Fluoreszenzbildes befindet sich im Strahlengang der Lichtquelle 11 lediglich das Filter 114, das ultraviolettes Licht durchläßt und sichtbares Licht blockiert. Im Strahlengang der Kamera befindet sich das für die Fluoreszenzlicht durchlässige Filter 123. Die Durchlaßbereiche der bei der Aufnahme eines Fluoreszenzbildes wirksamen Filter 114 und 123 sind so aufeinander abgestimmt, daß sich in dem Fluoreszenzwellenlängenbereich eine maximale Fluoreszenz ergibt. Bei einer Anregung durch ultraviolettes Licht im Bereich zwischen 340 und 380 nm liegt der Wellenlängenbereich mit maximaler Ausbeute an Fluoreszenzstrahlung zwischen 450 und 490 nm (blau).

Das bei der Aufnahme des Fluoreszenzbildes erzeugte Videosignal wird über den Bildverarbeitungsprozessor 25 dem Bildspeicher 22 zugeführt. Dabei werden noch Korrekturen durchgeführt, beispielsweise die Dunkelströme der Fernsehkamera kompensiert usw. Die in dem digitalen Bildspeicher 22 enthaltenen Werte F(x,y) stellen somit die Intensität des Fluoreszenzlichtes in den verschiedenen Bildpunkten des zweidimensionalen Untersuchungsbereiches 4 dar. Zur Verminderung des Rauschens können die während mehrerer Bildperioden entstehenden Bildsignale in dem Speicher 22 aufaddiert bzw. gemittelt werden.

In Fig. 2a ist das Intensitätsprofil F(x) längs einer durch ein Melanom in x-Richtung verlaufenden Linie mit F(x) dargestellt. Das Profil F(x) zeigt zwei relative Intensitätsmaxima F1, F2, die einem einige Millimeter breiten Übergangsbereich um das Melanom herum zuzuordnen sind. In diesem Bereich ergibt sich eine erhöhte Fluoreszenzintensität. Da die Fluoreszenzintensität über die übrigen, gesunden Flächen jedoch nicht konstant ist - beispielsweise weil die einzelnen Bezirke des Untersuchungsbereichs eine unterschiedliche Neigung zur Beleuchtungseinrichtung 11 haben und deshalb unterschiedlich stark beleuchtet werden - gibt es Werte F(x), die einer noch größeren Fluoreszenzintensität entsprechen. Aus diesem Grund ist es nicht ohne weiteres möglich, in dem Fluoreszenzbild die relativen Intensitätsmaxima zu bestimmen und damit ein Pigment-Muttermal der Haut eindeutig als Melanom zu identifizieren.

Daher wird noch ein Referenzbild erzeugt. Das Referenzbild soll den gleichen Einflüssen unterworfen sein wie das Fluoreszenzbild, jedoch soll die erhöhte Fluoreszenzaktivität am Rand des Melanoms nicht im Referenzbild erscheinen. Der Wellenlängenbereich, in dem der Untersuchungsbereich 4 beleuchtet wird und in dem das Referenzbild aufgenommen werden, müssen dabei übereinstimmen.

Beim Ausführungsbeispiel wurde ein Wellenlängenbereich um 435 nm gewählt. Dieser Bereich kann aber auch so gewählt sein, daß er mit dem Fluoreszenzwellenlängenbereich (450 bis 490 mm) übereinstimmt - falls die Beleuchtungseinrichtung in diesem Bereich eine hinreichende Intensität hat. In diesem Fall kann das Filter 124 entfallen und das Filter 123 ständig im Strahlengang der Kamera sein. Es ist aber auch möglich, das Referenzbild bei noch größeren Wellenlängen aufzunehmen. Bevorzugt wird als Wellenlängenbereich für das Referenzbild einer der sogenannten isobestischen Wellenlängenbereiche gewählt. Das sind die Bereiche, bei denen sauerstoffreiches und sauerstoffarmes Blut die gleiche Absorption zeigen. Das Referenzbild ist dann unabhängig vom Sauerstoffsättigungsgrad des Blutes, das die Färbung des Gewebes wesentlich mitbestimmt. Derartige Wellenlängenbereiche liegen u.a. bei ca. 550 nm oder bei 585 nm; in diesen Bereichen zeigt die UV-Lichtquelle 110 ausgeprägte (Quecksilber-)Emmissionslinien.

Das dem Referenzbild entsprechende elektrische Signal wird ebenfalls vom Analog-Digital-Wandler 21 digitalisiert, vom Bildverarbeitungsprozessor jedoch - gegebenenfalls unter Durchführung geeigneter Korrekturalgorithmen - in dem Speicher 23 gespeichert. Dieser enthält somit digitale Werte R(x,y), die der Helligkeit an den einzelnen Punkten des Untersuchungsbereichs entsprechen.

Fig. 2a zeigt den Verlauf R(x) des Intensitätsprofils für dieselbe Linie wie das Intensitätsprofil F(x) im Fluoreszenzbild. Man erkennt, daß das Signal R(x) einen entsprechenden Verlauf, wie das Signal F(x) hat, wenn man einmal von den relativen Maxima F1, F2 absieht, die in dem Profil R(x) keine Entsprechung haben. Diese Übereinstimmungen rühren daher, daß der Untersuchungsbereich bei beiden Bildern aus derselben Perspektive beleuchtet und aufgenommen wurde. Wenn dann noch dafür gesorgt wird, daß die Bilder in dichtem zeitlichen Abstand voneinander erfolgen, was ohne weiteres möglich ist, da nur Filter im Strahlengang gewechselt werden müssen, werden auch die Artefakte vermieden, die durch Bewegungen des Patienten in der Zeit zwischen der Aufnahme der Bilder hervorgerufen werden.

Nachdem auf diese Weise in den Bildspeichern 22 und 23 das Fluoreszenzbild und das Referenzbild gespeichert sind, wird aus den gespeicherten Werten ein Ausgangsbild A(x,y) abgeleitet, indem F(x,y) auf R(x,y) normiert wird. Zu diesem Zweck wird der Quotient, der demselben Bildpunkt zugeordneten Werte F(x,y) und R(x,y) gebildet und - gegebenenfalls nach einem zusätzlichen Verarbeitungsschritt - in dem Speicher 24 als Ausgangsbild A(x,y) gespeichert. Da die gesunden Hautpartien im Fluoreszenzbild und im Referenzbild zumindest annähernd gleichartige Intensitätsverläufe zeigen, hat das Ausgangsbild für die zugehörigen Bildpunkte einen zumindest annähernd konstanten Wert, der unterhalb eines in Fig. 2b gestrichelt angedeuteten Wertes Ao liegt. An den Stellen der relativen Maxima F1, F2 ergeben sich dagegen Maxima A1 und A2, die deutlich größer sind als dieser Wert.

In dem auf diese Weise erzeugten und auf dem Monitor 3 wiedergegebenen Ausgangsbild ist also der das Melanom einschließende Übergangsbereich 30 zwischen gesundem und kranken Gewebe heller als die gesunden Hautpartien. Jedoch können die Helligkeitsunterschiede so klein sein, daß sie einem Betrachter nicht sofort ins Auge fallen. In diesem Fall läßt sich der Kontrast zwischen gesundem Gewebe und dem Umgebungsbereich in einfacher Weise dadurch erhöhen, daß z.B. diejenigen Werte A(x,y), die den Schwellwert Ao überschreiten, mit einem Faktor multipliziert werden, der größer ist als 1, wodurch die betreffenden Bildpunkte auf dem Bildschirm des Monitors heller wiedergegeben werden, oder daß die Werte pseudofarbig dargestellt werden.

Da der Untersuchungsbereich relativ ausgedehnt sein kann, z.B. 15 x 20 cm, ist es möglich, größere Hautpartien schnell auf Melanome zu untersuchen.

Im Ausführungsbeispiel sind drei Bildspeicher - für das Fluoreszenzbild, das Referenzbild und das Ausbild vorgesehen, jedoch kann man mit zwei Bildspeichern auskommen, wenn z.B. in dem Speicher 22 das Bild F(x,y) durch das von dem Quotienten F(x,y)/R(x,y) abhängige Bild A(x,y) Bildpunkt für Bildpunkt ersetzt wird. Wenn die Quotientenbildung on-line beim Eingehen der Werte R(x,y) durchgeführt werden kann, ist sogar nur ein Bildspeicher erforderlich.

Im vorstehenden wurde das erfindungsgemäße Verfahren im Zusammenhang mit der Diagnose von Melanomen beschrieben. Es ist jedoch auch möglich, damit andere Tumoren an der Oberfläche des menschlichen Körpers zu entdecken, deren Fluorenszenzlichtemission sich von derjenigen des gesunden Gewebes unterscheidet.

## Patentansprüche

1. Verfahren zur Erfassung von Anomalien der Haut, insbesondere von Melanomen, wobei das durch Beleuchtung mit ultraviolettem Licht aus einem ersten Wellenlängenbereich erzeugte Fluoreszenzlicht ausgewertet wird,
dadurch gekennzeichnet, daß ein zweidimensional ausgedehnter, aus einer Anzahl von Bildpunkten (x,y) bestehender Untersuchungsbereich (4) mit Licht des ersten Wellenlängenbereichs beleuchtet und ein Fluoreszenzbild (F(x,y)) des Untersuchungsbereichs aufgenommen wird, daß der Untersuchungsbereich mit Licht einer zweiten Wellenlänge beleuchtet wird und ein Referenzbild (R(x,y)) in dem zweiten Wellenlängenbereich aufgenommen wird und daß für die verschiedenen Bildpunkte (x,y) des Untersuchungsbereichs (4) der dem jeweiligen Bildpunkt zugeordnete Bildwert des Fluoreszenzbildes (F(x,y)) auf den diesem Bildpunkt zugeordneten Bildwert des Referenzbildes (R(x,y)) normiert wird und der normierte Wert dem entsprechenden Bildpunkt (x,y) in einem Ausgangsbild (A(x,y)) zugeordnet wird.

2. Vorrichtung zur Durchführung des Verfahrens nach Anspruch 1 mit,
wenigstens einer Lichtquelle (110) zur Beleuchtung des Untersuchungsbereichs (4) mit Licht in einem ersten und in einem zweiten Wellenlängenbereich, einer auf den Untersuchungsbereich ausrichtbaren Kamera (12) zum Aufnehmen des Fluoreszenzbildes und des Referenzbildes und zur Erzeugung entsprechender elektrischer Signale, einer Speicheranordnung (22,24) zum Speichern wenigstens eines Bildes und einer Verarbeitungseinheit (25) zur Erzeugung eines Ausgangsbildes (A(x,y)) in Abhängigkeit von dem Quotienten der denselben Bildpunkten zugeordneten Signalwerte des Fluoreszenz- und des Referenzbildes (F(x,y) / (R(x,y)).

3. Vorrichtung nach Anspruch 2,
gekennzeichnet durch mindestens eine sowohl im ersten als auch im zweiten Wellenlängenbereich emittierende Lichtquelle (110), ein erstes Filter (114) zum Durchlassen von Licht aus dem ersten Wellenlängenbereich und zum Unterdrücken von Licht mit der Wellenlänge des Fluoreszenzlichts sowie durch ein zweites Filter (113) zum Durchlassen des zweiten Wellenlängenbereiches, wobei jeweils eines der beiden Filter in den Strahlengang zwischen der Lichtquelle und dem Untersuchungsbereich (4) anordbar ist.

4. Vorrichtung nach Anspruch 2 oder 3,
gekennzeichnet durch eine im Strahlengang der Lichtquelle befindliche Mattscheibe (112).

5. Vorrichtung nach einem der Ansprüche 2 bis 4,
gekennzeichnet durch ein vor der Kamera anordbares Filter (113) zur Unterdrückung von Licht aus dem ersten Wellenlängenbereich und zum Durchlassen von Licht aus dem Wellenlängenbereich des Fluoreszenzlichts.

6. Vorrichtung nach einem der Ansprüche 2 bis 5,
gekennzeichnet durch ein vor der Kamera anordbares Filter (124) zum Durchlassen von Licht aus dem zweiten Wellenlängenbereich.

7. Vorrichtung nach einem der Ansprüche 2 bis 6,
gekennzeichnet durch in den Strahlengang vor der Kamera (12) und der Lichtquelle (110) einführbare Polarisationsfilter (115,121) , die so beschaffen und angeordnet sind, daß die gerichtete Reflexion in dem von der Kamera erzeugten Bild unterdrückt wird.

8. Vorrichtung nach einem der vorhergehenden Ansprüche,
dadurch gekennzeichnet, daß der zweite Wellenlängenbereich ein isobestischer Wellenlängenbereich ist.

## Claims

1. A method of detecting anomalies of the skin, particularly melanomas, in which the fluorescence light produced by illumination with light from a first wavelength range is evaluated, characterized in that a two-dimensional examination region (4), consisting of a number of pixels (x,y), is illuminated with light of the first wavelength range and a fluorescence image (F(x,y)) of the examination region is recorded, that the examination region is illuminated with light of a second wavelength range and a reference image (R(x,y)) is recorded in the second wavelength range, and that for the various pixels (x,y) of the examination region (4) the image value of the fluorescence image F(x,y) associated with the relevant pixel is standardized to the image value of the reference image (R(x,y)) associated with the relevant pixel, the standardized value being assigned to the corresponding pixel (x,y) in an output image (A(x,y)).

2. A device for carrying out the method claimed in Claim 1, comprising at least one light source (110) for illuminating the examination region (4) with tight in a first and in a second wavelength range, a camera (12) which can be directed onto the examination region in order to record the fluorescence image and the reference image and to produce corresponding electrical signals, a memory device (22, 24) for storing at least one image and a processing unit (25) for producing an output image (A(x,y)) in dependence upon the quotient of the signal values of the fluorescence image and of the reference image (F(x,y)/R(x,y)) assigned to the same pixels.

3. A device as claimed in Claim 2, characterized in that it comprises at least one light source (110) which emits both in the first and in the second wavelength range, a first filter (114) for transmitting light from the first wavelength range and for suppressing light having the wavelength of the fluorescence light, and a second filter (113) for transmitting the second wavelength range, it being possible to arrange each time one of the two filters in the beam path between the light source and the examination region (4).

4. A device as claimed in Claim 2 or 3, characterized in that it comprises a frosted pane (112) which is present in the beam path of the light source.

5. A device as claimed in any one of Claims 2 to 4, characterized in that it comprises a filter (113) which can be arranged in front of the camera in order to suppress light from the first wavelength range and to transmit light from the wavelength range of the fluorescence light.

6. A device as claimed in any one of Claims 2 to 5, characterized in that it comprises a filter (124) which can be arranged in front of the camera in order to transmit light from the second wavelength range.

7. A device as claimed in any one of Claims 2 to 6, characterized in that it comprises polarization filters (115, 121) which can be introduced into the beam path in front of the camera (12) and the light source (110) and are constructed and arranged so that the directed reflection in the image produced by the camera is suppressed.

8. A device as claimed in any one of the preceding Claims, characterized in that the second wavelength range is an isobestic wavelength range.

## Revendications

1. Procédé de détection d'anomalies de la peau, en particulier de mélanomes, la lumière fluorescente produite par l'exposition à une lumière ultraviolette à partir d'une première plage de longueurs d'ondes étant analysée, caractérisé en ce qu'une région à examiner (4) étendue bidilmensionnelle composée d'un nombre de pixels (x, y) est exposée à la lumière de la première plage de longueurs d'ondes et une image fluorescente (F(x,y)) de la région à examiner est enregistrée, que la région à examiner est exposée à la lumière d'une deuxième plage de longueurs d'ondes et qu'une image de référence (R(x,y)) est enregistrée dans la deuxième plage de longueurs d'ondes et que, pour les différents pixels (x, y) de la région à examiner (4), la valeur d'image affectée au pixel respectif de l'image fluorescente (F(x,y)) est normalisée sur la valeur d'image de l'image de référence (R(x,y)) respectivement affectée à ce pixel et la valeur normalisée est affectée au pixel correspondant (x, y) dans une image de sortie (A(x,y)).

2. Dispositif d'exécution du procédé selon la revendication 1 avec au moins une source lumineuse (110) pour l'exposition de la région à examiner (4) avec une lumière dans une première et une deuxième plages de longueurs d'ondes, une caméra (12) orientable sur la région à examiner pour l'enregistrement de l'image fluorescente et de l'image de référence et pour la production de signaux électriques correspondants, d'un dispositif de mémoire (22, 24) pour la sauvegarde d'au moins une image et une unité de traitement (25) pour la production d'une image de sortie (A(x,y)) en fonction du quotient des valeurs de signal du signal de fluorescence et du signal de référence affectées aux mêmes pixels (F(x,y)/R(x, y).

3. Dispositif selon la revendication 2, caractérisé par au moins une source lumineuse (110) émettant à la fois dans la première et dans la deuxième plages de longueurs d'ondes, par un premier filtre (114) pour le passage de lumière à partir de la première plage de longueurs d'ondes et pour la suppression de la lumière avec la longueur d'onde de la lumière fluorescente ainsi que par un deuxième filtre (113) pour le passage de la deuxième plage de longueurs d'ondes, l'un des deux filtres pouvant être disposé dans le trajet des rayons entre la source lumineuse et la région à examiner (4).

4. Dispositif selon l'une des revendications 2 ou 3, caractérisé par un écran dépoli (112) se trouvant dans le trajet des rayons de la source lumineuse.

5. Dispositif selon l'une des revendications 2 à 4, caractérisé par un filtre (113) à disposer devant la caméra pour supprimer la lumière provenant de la première plage de longueurs d'ondes et pour le passage de la lumière à partir de la plage de longueurs d'onde de la lumière fluorescente.

6. Dispositif selon l'une des revendications 2 à 5, caractérisé par un filtre (114) à disposer devant la caméra pour le passage de lumière à partir de la deuxième plage de longueurs d'ondes.

7. Dispositif selon l'une des revendications 2 à 6, caractérisé par des filtres de polarisation (115, 121) à insérer dans le trajets de rayons devant la caméra (12) et la source lumineuse (11) qui sont conçus et disposés de telle manière que la réflexion dirigée soit supprimée dans l'image produite par la caméra.

8. Dispositif selon l'une des revendications précédentes, caractérisé en ce que la deuxième plage de longueurs d'ondes est une plage de longueurs d'ondes isobestique.
